(19) Europäisches Patentamt
European Patent Office
Office européen des brevets

(11) Veröffentlichungsnummer: **0 278 467 B1**

# (12) EUROPÄISCHE PATENTSCHRIFT

(45) Veröffentlichungstag der Patentschrift: **11.12.91**

(51) Int. Cl.[5]: **A61K 31/505**

(21) Anmeldenummer: **88101831.1**

(22) Anmeldetag: **09.02.88**

## (54) Verwendung von Oxochinazolinderivaten bei der Behandlung von Hyperurikämie.

(30) Priorität: **11.02.87 DE 3704203**

(43) Veröffentlichungstag der Anmeldung:
**17.08.88 Patentblatt 88/33**

(45) Bekanntmachung des Hinweises auf die Patenterteilung:
**11.12.91 Patentblatt 91/50**

(84) Benannte Vertragsstaaten:
**AT BE CH DE ES FR GB IT LI LU NL SE**

(56) Entgegenhaltungen:
**EP-A- 0 113 911**
**DE-A- 2 557 425**

**ARZNEIMITTEL-FORSCHUNG/DRUG RESEARCH, Band 36(II), Nr. II, 1986, Seiten 1637-1641; K. TSURUMI et al.: "Effect of the new antiallergic drug 11-Oxo-11H-pyrido[2,1-b] quinazoline-2-carboxylic acid on inflammatory reactions and platelet aggregation"**

**ARTHRITIS AND RHEUMATISM, Band 18, Nr. 6, November 1975, Seiten 847-851; S.L. WALLACE: "Colchicine and new antiinflammatory drugs for the treatment of acute gout"**

(73) Patentinhaber: **BOEHRINGER INGELHEIM KG**
**Postfach 200**
**W-6507 Ingelheim am Rhein(DE)**
(84) Benannte Vertragsstaaten:
**BE CH DE ES FR IT LI LU NL SE AT**

Patentinhaber: **BOEHRINGER INGELHEIM INTERNATIONAL GmbH**
**Postfach 20**
**W-6507 Ingelheim am Rhein(DE)**
(84) Benannte Vertragsstaaten:
**GB**

(72) Erfinder: **Rauber, Gerhard, Dr.**
**Magdeburger Strasse 32**
**W-6507 Ingelheim(DE)**
Erfinder: **Stechert, Roland**
**Dr. Johannes Kohl Strasse 16**
**W-6530 Bingen am Rhein(DE)**

**EP 0 278 467 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung bestimmter bekannter Oxochinazolinderivate bei der Behandlung der Hyperurikämie.

Die Hyperurikämie stellt die pathophysiologische Ursache der Gicht dar. Die derzeit zur Behandlung der Hyperurikämie zur Verfügung stehenden Medikamente weisen zwar eine gute Wirksamkeit auf, sind jedoch mit unterschiedlichen unerwünschten Wirkungen belastet. Es handelt sich entweder um Urikosurika (Mittel, die die Ausscheidung der Harnsäure erhöhen, wie z.B. Probenecid, Sulfinpyrazon, Benzbromaron) oder um Urikostatika (Mittel, die die Synthese der Harnsäure hemmen, wie Allopurinol).

Das Nebenwirkungsprofil der Präparate im einzelnen:

- Bei Probenecid können bei 10% der Patienten Magen-Darm-Störungen und in 4 % der Fälle allergische Hautreaktionen auftreten, in Einzelfällen wurde auch ein nephrotisches Syndrom (Erkrankung der Niere) beschrieben.
- Sulfinpyrazon wirkt gleichzeitig als Thrombozyten-Aggregationshemmer und kann ebenfalls zu Magen-Darm-Störungen führen. Des weiteren wird Natriumretention (führt zur Wassereinlagerung) und in Einzelfällen auch eine Agranulozytose (Schädigung des blutbildenden Systems) beschrieben.
- Unter Benzbromaron kann es ebenfalls zu Magen-Darm-Störungen bei der Anwendung kommen.
- Bei Allopurinol kann es neben Magen-Darm-Störungen ebenfalls zu unerwünschten Wirkungen am blutbildenden System kommen, die jedoch recht selten sind. Pruritus sowie Allergien treten relativ häufig auf, wobei in seltenen Fällen auch ein Lyell-Syndrom (Syndrom der verbrühten Haut) beschrieben ist.

Wie nun gefunden wurde, sind für die Behandlung der Hyperurikämie überraschenderweise die Verbindungen gemäß der Formel

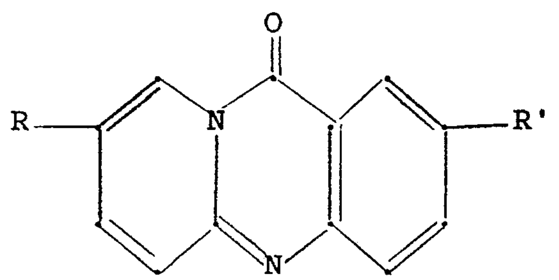

(I),

wobei für die Reste R und R' die nachstehenden Bedeutungspaare gelten (R jeweils zuerst genannt):

| | |
|---|---|
| H/COOH | COOH/H |
| $CH_3$/COOH | COOH/$CH_3$ |
| $OCH_3$/COOH | COOH/$OCH_3$ |
| $CH(CH_3)_2$/COOH | COOH/$CH(CH_3)_2$ |
| H/Tetrazol-5-yl | Tetrazol-5-yl/H |
| H/$CONH_2$ | |
| H/CN | |

Die oben erwähnten Verbindungen sind bereits als Arzneistoffe beschrieben worden (DE-A-2557425). Als Anwendungsgebiete wurden angegeben: Prophylaxe und Behandlung allergischer Krankheiten wie Asthma, Heufieber, Conjunktivitis, Urticaria, Ekzeme, Dermatitis. Ferner wurde eine muskelrelaxierende (bronchodilatorische) und vasodilatorische Wirkung erwähnt.

Ein Hinweis auf eine harnsäuresenkende Wirkung findet sich nicht, und eine solche Wirkung wird durch die bekannten Wirkungen auch nicht nahegelegt.

Gegenüber den bekannten Mitteln zur Behandlung der Hyperurikämie bzw. Gicht haben die erfindungsgemäß verwendbaren Verbindungen, etwa die 11-Oxo-11-H-pyrido-[2,1-b]-chinazolin-2-carbonsäure und ihre Salze, den Vorzug besserer Verträglichkeit. So wurden bei der Behandlung von über 1000 Patienten mit dieser Verbindung in einem anderen Indikationsgebiet keinerlei präparatespezifischen Nebenwirkungen

festgestellt.

Für die Anwendung werden die Verbindungen in üblicher Weise zu gebräuchlichen galenischen Zubereitungen verarbeitet. Deutlich im Vordergrund steht die orale Anwendung, vorzugsweise in Form von Kapseln; aber auch alle anderen oralen Darreichungsformen kommen in Betracht, also beispielsweise Tabletten, Dragées, Granulate, Suspensionen, Retardformen.

Die Dosis pro Tag beträgt etwa 100 - 1000 mg; sie kann in 1 - 3 Einzeldosen verabreicht werden.

In einer Studie an 12 ambulanten Patienten mit einer symptomlosen Hyperurikämie erfolgte eine Behandlung mit 11-Oxo-11-H-pyrido[2,1-b]chinazolin-2-carbonsäure in Form von Tabletten mit üblichen Hilfsstoffen. Die Dosis betrug 3 mal 200 mg täglich über 2 Wochen mit einer anschließenden einwöchigen Wash-out-Phase. Vor Behandlung lagen die Harnsäurewerte im Serum im Mittel bei 8,56 ± 0,6 mg/100 ml. Diese Werte sanken nach zweiwöchiger Therapie auf 6,1 ± 0,8 mg/100 ml signifikant um 29 % ab. Nach der Wash-out-Phase erfolgte ein Wiederanstieg auf 7,7 mg ± 0,9 mg/100 ml.

In einer weiteren Studie wurden 8 stationäre Probanden kontinuierlich 8 Tage lang mit einer purinreichen Diät ernährt. Hierdurch wurde ein erhöhter Harnsäurespiegel im Serum erzeugt, der vom 3. Tag an stabil war. Ab dem 5. Tag erhielten die Probanden zusätzlich 3 x 200 mg/Tag der vorstehend genannten Verbindung als Tabletten. Am 4. Tag unter der Diät lagen die Harnsäurespiegel bei 7,9 ± 0,95 mg/100ml stabil im erhöhten Bereich. Nach Abschluß der Behandlung (4 Tage), d.h. am Ende des 8. Versuchstages, waren die Werte auf 5,1 ± 0,82 mg/100 ml signifikant um 35 % gefallen. Auch hier wurde also ein eindeutig harnsäuresenkender Effekt der Verbindung festgestellt.

Formulierungsbeispiele

1. Tabletten

| | |
|---|---|
| 11-Oxo-11-H-pyrido[2,1-b]-chinazolin-2-carbonsäure | 100 g |
| kolloidale Kieselsäure | 10 g |
| Milchzucker | 118 g |
| Kartoffelstärke | 60 g |
| Polyvinylpyrrolidon | 6 g |
| Natrium-Celluloseglykolat | 4 g |
| Magnesiumstearat | 2 g |

Die Bestandteile werden in üblicher Weise zu Tabletten von 300 mg verarbeitet.

2. Kapseln

| | |
|---|---|
| Wirkstoff nach Formel I | 300 g |
| Maisstärke | 100 g |

Die Bestandteile werden gut vermischt und die Mischung in 400 mg-Portionen in Gelatine-Steckkapseln abgefüllt.

**Patentansprüche**

**1.** Verwendung von Verbindungen der Formel

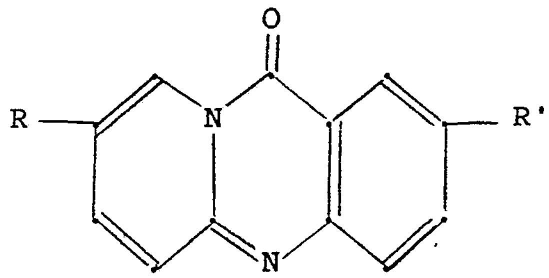

(I),

wobei für die Reste R und R' die nachstehenden Bedeutungspaare gelten (R jeweil zuerst genannt):

| | |
|---|---|
| H/COOH | COOH/H |
| $CH_3$/COOH | COOH/$CH_3$ |
| $OCH_3$/COOH | COOH/$OCH_3$ |
| $CH(CH_3)_2$/COOH | COOH/$CH(CH_3)_2$ |
| H/Tetrazol-5-yl | Tetrazol-5-yl/H |
| H/$CONH_2$ | |
| H/CN | |

zur Herstellung von Arzneimitteln für die Behandlung der Hyperurikämie.

2. Verwendung der 11-Oxo-11-H-pyrido[2,1-b]-chinazolin-2-carbonsäure oder ihrer Salze zur Herstellung von Arzneimitteln für die Behandlung der Hyperurikämie.

## Claims

1. Use of compounds of formula

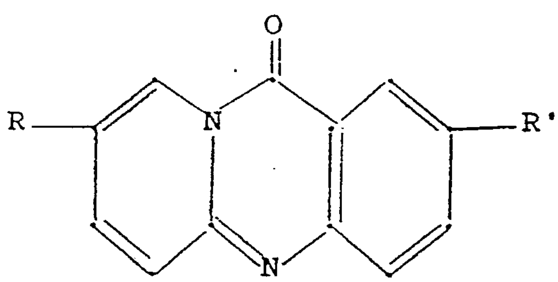

(I)

whilst the groups R and R' have the following pairs of definitions (R being mentioned first in each case):

| | |
|---|---|
| H/COOH | COOH/H |
| $CH_3$/COOH | COOH/$CH_3$ |
| $OCH_3$/COOH | COOH/$OCH_3$ |
| $CH(CH_3)_2$/COOH | COOH/$CH(CH_3)_2$ |
| H/tetrazol-5-yl | tetrazol-5-yl/H |
| H/$CONH_2$ | |
| H/CN | |

for preparing pharmaceutical compositions for the treatment of hyperuricaemia.

2. Use of 11-oxo-11-H-pyrido[2,1-b]quinazolin-2-carboxylic acid or the salts thereof in the treatment of hyperuricaemia.

**Revendications**

1. Utilisation de composés de formule

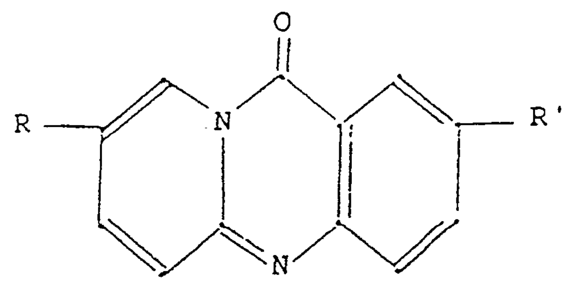

(I),

dans laquelle les restes R et R' ont les significations suivantes (R est nommé à chaque fois en premier) :

| | |
|---|---|
| H/COOH<br>$CH_3$/COOH<br>$OCH_3$/COOH<br>$CH(CH_3)_2$/COOH<br>H/5-tétrazolyle<br>H/$CONH_2$<br>H/CN | COOH/H<br>COOH/$CH_3$<br>COOH/$OCH_3$<br>COOH/$CH(CH_3)_2$<br>5-tétrazolyle/H |

pour la préparation de médicaments pour le traitement de l'hyperuricémie.

2. Utilisation de l'acide 11-oxo-11-H-pyrido[2,1-b]-quinazoline-2-carboxylique ou de ses sels pour la préparation de médicaments pour le traitement de l'hyperuricémie.